# EUROPEAN PATENT APPLICATION

(11) **EP 4 357 464 A1**
(43) Date of publication of application: **24.04.2024**
(21) Application number: 23204675.5
(22) Date of filing: 19.10.2023
(51) Int. Cl.: C12Q 1/70

(54) **A METHOD FOR DETECTING THE PRESENCE OF A DENGUE VIRUS SEROTYPE IN A SAMPLE CONTAINING AT LEAST ONE DENGUE VIRUS SEROTYPE**

(30) Priority: 20.10.2022 EP 22202665
(71) Applicant: Takeda Vaccines, Inc., Cambridge, MA 02139 (US)
(72) Inventor: THOMSON, Andrew Montgomery, 138567 Singapore (SG)
(74) Representative: Maiwald GmbH

(57) **Abstract**

The present invention provides methods and kits for detecting the presence of a dengue virus serotype in a sample as well as uses of these methods and kits in quality control of one or more vaccines containing live, attenuated dengue virus and diagnosis of a dengue virus infection in a patient sample. Further provided are quality control methods for vaccines containing live, attenuated dengue virus.

## Description

### Co-filed sequence listing

The present specification makes reference to a sequence listing (submitted electronically on the same date as the present application). The entire contents of the Sequence Listing are incorporated herein by reference.

### Technical field

The present invention relates to methods and kits for detecting the presence of a dengue virus serotype in a sample as well as uses of these methods and kits in quality control of one or more vaccines containing live, attenuated dengue virus and diagnosis of a dengue virus infection in a patient sample. Further provided are quality control methods for vaccines containing live, attenuated dengue virus.

### Background of the Invention

Dengue virus is an arbovirus in the *Flaviviridae* family. It is an enveloped virus with an isometric nucleocapsid, consisting of capsid proteins and a linear positive sense RNA genome. Dengue viruses are endemic in most regions of the tropics and are transmitted by mosquitoes, typically *Aedes aegypti*, causing symptomatic infections or asymptomatic seroconversion. Symptomatic dengue infection is a systemic and dynamic disease. It has a wide clinical spectrum that includes both severe and non-severe clinical manifestations. There are four different dengue serotypes (DENV-1 to DENV-4) which are genetically related to yellow fever and similar tick-borne encephalitis viruses.

One tetravalent dengue vaccine (TDV) tested in clinical trials and now approved as the product QDENGA^{®} consists of all four dengue serotypes, TDV-1, TDV-2, TDV-3 and TDV-4. TDV-2 is a live attenuated recombinant virus that originated from PDK-53 that became non-pathogenic after continuous passaging of DENV-2 virus in primary dog kidney (PDK) cells. The mutations necessary and sufficient for the attenuated phenotype of TDV-2 virus have been genetically identified (Butrapet et al., J. Virol., vol. 74, no. 7 (2000), 3011-3019). These mutations are a C-to-T mutation at genomic nucleotide position 57 in the 5' noncoding region, a G-to-A mutation at genomic nucleotide position 2579 (causing a Gly-to-Asp mutation at amino acid position 53 in the nonstructural protein (NS) 1) and an A-to-T mutation at genomic nucleotide position 5270 (causing a Glu-to-Val mutation at amino acid position 250 in the nonstructural protein (NS) 3. These three genetic determinants of attenuation reside outside of the structural gene regions of the TDV-2 viral genome. TDV-1, TDV-3 and TDV-4 are chimeric variants having the backbone of Dengue virus 2 PDK53 and the prM and E genes of Dengue virus 1, Dengue virus 3 and Dengue virus 4, respectively, which maintain the attenuation phenotype of TDV-2.

For quality control of the monovalent drug substance comprising a single live, attenuated dengue virus and the tetravalent drug product comprising a mixture of all four live, attenuated viruses it is essential to confirm the presence of the dengue virus serotype(s) in the vaccine formulation before batches are released for administration to individuals.

In view thereof, there is a need for detecting the presence of the dengue serotypes in the monovalent drug substance and in the tetravalent drug product. The detection method should be both specific and robust with respect to the presence of additional constituents.

### Summary of the Invention

The technical problems underlying the invention are solved by the provision of the subject-matter as defined in the claims.

According to a first aspect, a method for detecting the presence of a dengue virus serotype in a sample containing at least one dengue virus serotype comprising dengue virus nucleic acids is provided comprising the steps:
a) optionally purifying the dengue virus nucleic acids from said sample;
b) preparing double-stranded DNA templates from said dengue virus nucleic acids;
c) amplifying said double-stranded DNA templates of step b) by polymerase chain reaction (PCR) using a DNA polymerase with a primer pair selected from the group consisting of
   (i) a dengue serotype 1 (DENV-1) specific primer pair, wherein the forward primer comprises the nucleotide sequence set forth in SEQ ID NO: 1 or a variant thereof having at least 85% sequence identity thereof and the reverse primer comprises the nucleotide sequence set forth in SEQ ID NO: 2 or a variant thereof having at least 85% sequence identity thereof such that in the presence of the DENV-1 serotype an amplicon having a length of about 400 bp to about 500 bp generated;
   (ii) a dengue serotype 2 (DENV-2) specific pair, wherein the forward primer comprises the nucleotide sequence set forth in SEQ ID NO: 3 or a variant thereof having at least 85% sequence identity thereof and the reverse primer comprises the nucleotide sequence set forth in SEQ ID NO: 4 or a variant thereof having at least 85% sequence identity thereof such that in the presence of the DENV-2 serotype an amplicon having a length of about 300 bp to about 400 bp is generated;
   (iii) a dengue serotype 3 (DENV-3) specific primer pair, wherein the forward primer comprises the nucleotide sequence set forth in SEQ ID NO: 5 or a variant thereof having at least 85% sequence identity thereof and the reverse primer comprises the nucleotide sequence set forth in SEQ ID NO: 6 or a variant thereof having at least 85% sequence identity thereof such that in the presence of the DENV-3 serotype an amplicon having a length of about 700 bp to about 800 bp is generated; and/or
   (iv) a dengue serotype 4 (DENV-4) specific primer pair, wherein the forward primer comprises the nucleotide sequence set forth in SEQ ID NO: 7 or a variant thereof having at least 85% sequence identity thereof and the reverse primer comprises the nucleotide sequence set forth in SEQ ID NO: 8 or a variant thereof having at least 85% sequence identity thereof such that in the presence of DENV-4 serotype an amplicon having a length of about 200 bp to about 300 bp is generated;
d) separating the generated amplicon(s); and
e) detecting the generated amplicon(s), wherein the presence of an amplicon having a length of about 400 bp to about 500 bp is indicative for the presence of DENV-1, the presence of an amplicon having a length of about 300 bp to about 400 bp is indicative for the presence of DENV-2, the presence of an amplicon having a length of about 700 bp to about 800 bp is indicative for the presence of DENV-3 and/or the presence of an amplicon having a length of about 200 bp to about 300 bp is indicative for the presence of DENV-4.

The inventors have surprisingly found that the above RT-PCR method is specific for the detection of dengue virus serotypes in pharmaceutical formulations. In particular, it is suitable for the detection of the live, attenuated TDV-2 virus strain and the chimeric live, attenuated TDV-1, TDV-3 and TDV-4 virus strains. The primers in the inventive method do not generate unspecific fragments in contrast to a comparative experiment wherein different primer pairs have generated unspecific fragments which may interfere with or hinder the correct interpretation of the results.

According to a second aspect, the use of the inventive method in the quality control of vaccines containing live, attenuated dengue virus is provided.

According to a third aspect, the use of the inventive method in the diagnosis of a dengue virus infection in a patient sample is provided.

According to a fourth aspect, a kit for detecting the presence of dengue virus serotype in a sample is provided containing at least one dengue virus comprising
(i) a dengue serotype 1 (DENV-1) specific primer pair, wherein the forward primer comprises the nucleotide sequence set forth in SEQ ID NO: 1 or a variant thereof having at least 85% sequence identity thereof and the reverse primer comprises the nucleotide sequence set forth in SEQ ID NO: 2 or a variant thereof having at least 85% sequence identity thereof;
(ii) a dengue serotype 2 (DENV-2) specific pair, wherein the forward primer comprises the nucleotide sequence set forth in SEQ ID NO: 3 or a variant thereof having at least 85% sequence identity thereof and the reverse primer comprises the nucleotide sequence set forth in SEQ ID NO: 4 or a variant thereof having at least 85% sequence identity thereof;
(iii) a dengue serotype 3 (DENV-3) specific primer pair, wherein the forward primer comprises the nucleotide sequence set forth in SEQ ID NO: 5 or a variant thereof having at least 85% sequence identity thereof and the reverse primer comprises the nucleotide sequence set forth in SEQ ID NO: 6 or a variant thereof having at least 85% sequence identity thereof; and/or
(iv) a dengue serotype 4 (DENV-4) specific primer pair, wherein the forward primer comprises the nucleotide sequence set forth in SEQ ID NO: 7 or a variant thereof having at least 85% sequence identity thereof and the reverse primer comprises the nucleotide sequence set forth in SEQ ID NO: 8 or a variant thereof having at least 85% sequence identity thereof.

According to a fourth aspect, the present invention provides the use of any of the inventive kits according to the third aspect of the invention in the quality control of vaccines containing live, attenuated dengue virus.

According to a fifth aspect, the present invention provides the use of any of the inventive kits according to the third aspect of the invention in the diagnosis of a virus infection in a patient sample.

According to a sixth aspect, a quality control method for vaccines comprising performing the inventive method and at least one further method selected from the group consisting of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s) is provided.

### Brief Description of the Drawings

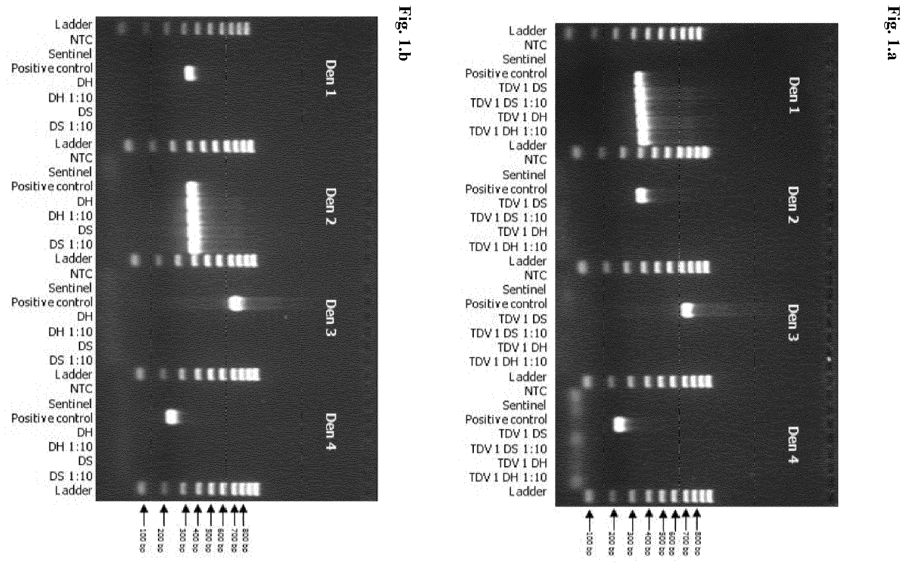

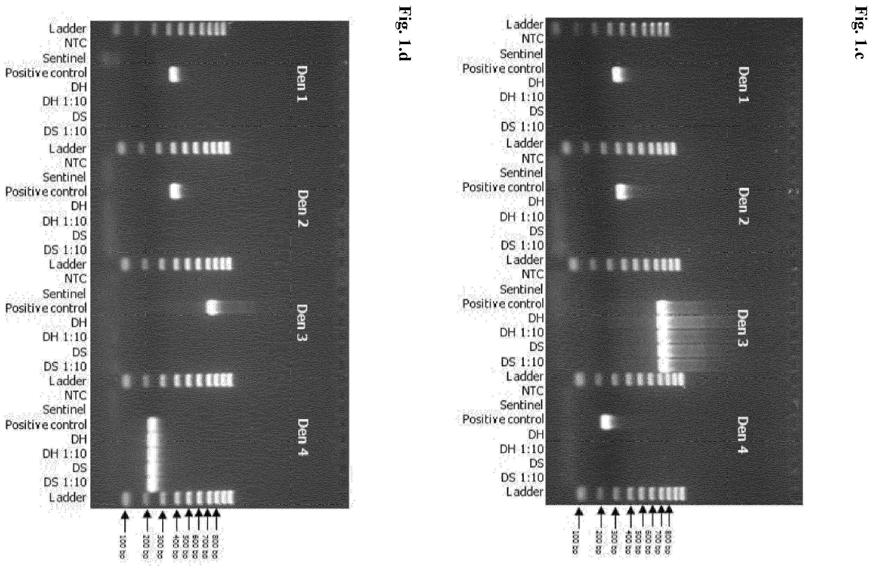

**Figure 1****.a** shows the scan of RT-PCR Fragments of TDV-1 DS / TDV-1 Daily Harvest (DH). Ladder: molecular weight ladder; NTC: no template control; Sentinel: Extraction control; TDV DS 1: 10: drug substance diluted 1: 10 before RNA extraction; TDV DH: Daily harvest. Den 1: PCR with TDV-1 specific primer set; Den 2: PCR with TDV-2 specific primer set; Den 3: PCR with TDV-3 specific primer set; Den 4: PCR with TDV-4 specific primer set.
**Figure 1****.b** shows the scan of RT-PCR Fragments of TDV-2 DS / TDV-2 Daily Harvest (DH). Ladder: molecular weight ladder; NTC: no template control; Sentinel: Extraction control; TDV DS 1: 10: drug substance diluted 1: 10 before RNA extraction; TDV DH: Daily harvest. Den 1: PCR with TDV-1 specific primer set; Den 2: PCR with TDV-2 specific primer set; Den 3: PCR with TDV-3 specific primer set; Den 4: PCR with TDV-4 specific primer set.
**Figure 1****.c** shows the scan of RT-PCR Fragments of TDV-3 DS / TDV-3 Daily Harvest (DH). Ladder: molecular weight ladder; NTC: no template control; Sentinel: Extraction control; TDV DS 1: 10: drug substance diluted 1: 10 before RNA extraction; TDV DH: Daily harvest. Den 1: PCR with TDV-1 specific primer set; Den 2: PCR with TDV-2 specific primer set; Den 3: PCR with TDV-3 specific primer set; Den 4: PCR with TDV-4 specific primer set.
**Figure 1****.d** shows the scan of RT-PCR Fragments of TDV-4 DS / TDV-4 Daily Harvest (DH). Ladder: molecular weight ladder; NTC: no template control; Sentinel: Extraction control; TDV DS 1: 10: drug substance diluted 1: 10 before RNA extraction; TDV DH: Daily harvest. Den 1: PCR with TDV-1 specific primer set; Den 2: PCR with TDV-2 specific primer set; Den 3: PCR with TDV-3 specific primer set; Den 4: PCR with TDV-4 specific primer set.

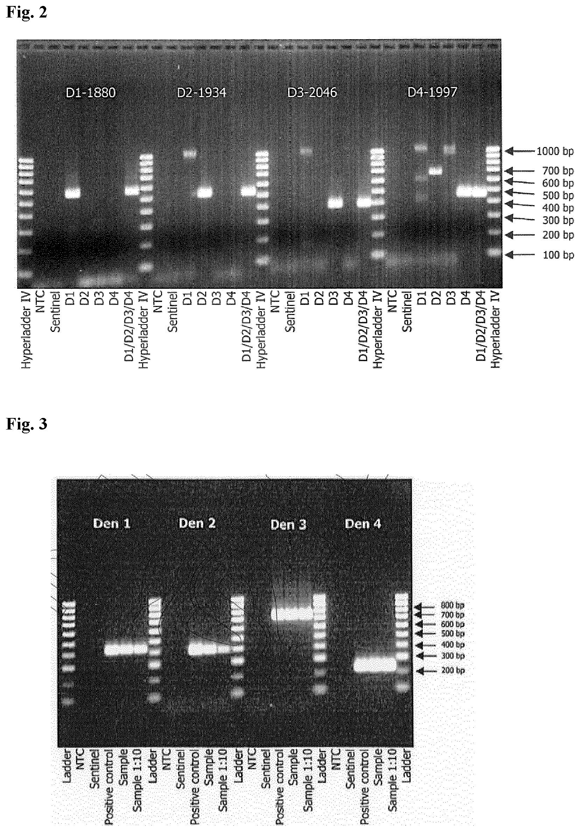

**Figure 2** shows the scan of RT-PCR fragments obtained according to a comparative method using primer pairs other than the inventive primer pairs. Hyperladder IV: molecular weight ladder; NTC: no template control; Sentinel: Extraction control; D1: TDV-1; D2: TDV-2; D3: TDV-3; D4: TDV-4; D1-1880: PCR with D1-1880 as reverse primer; D2-1934: PCR with D2-1934 as reverse primer; D3-2046: PCR with D3-2046 as reverse primer; D4-1997: PCR with D4-1997 as reverse primer.
**Figure 3** shows the analysis of samples including the tetravalent drug product by the identity assay according to the invention.

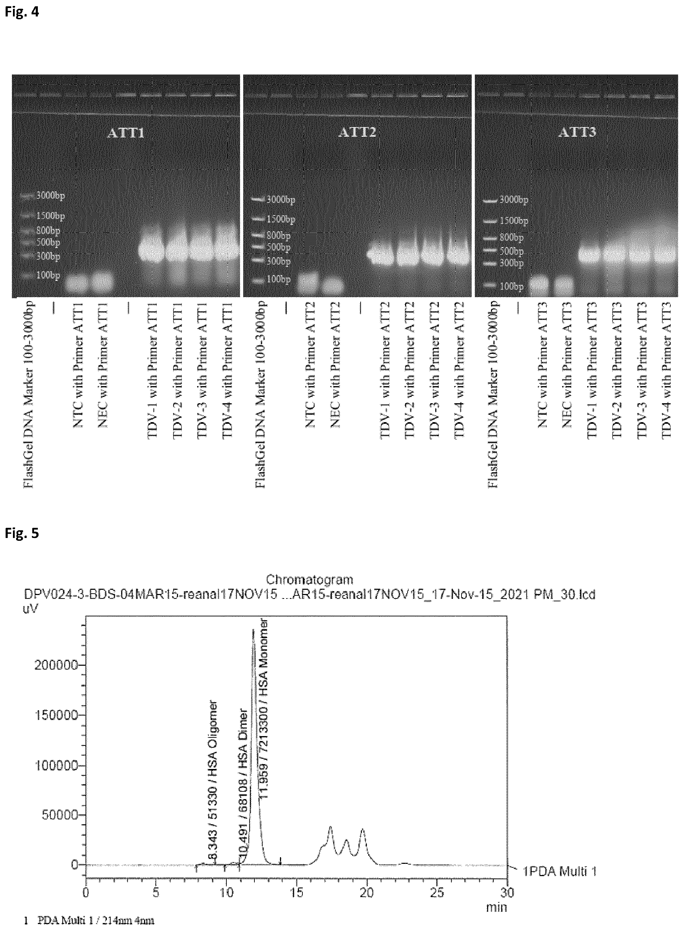

**Figure 4** FlashGel photos of the RT-PCR products (ATT1, ATT2 and ATT3) for the demonstration of the specificity for TDV-1, -2, -3 and -4, with the Primer pairs: ATT1 (324 bp), ATT2 (333 bp) and ATT3 (408 bp). The negative controls were negative, only the residual primers resp. primer dimers (1 primer >50 nt) are visible. ATT stands for attenuated and refers to the three attenuation sites ATT1, ATT2 and ATT3.
**Figure 5** shows the chromatogram of HSA in TDV (Takeda's tetravalent Dengue Vaccine) sample (DPV024-3 BDS) [bulk drug substance] .

### Detailed Description of the Invention

Where the term "comprise" or "comprising" is used in the present description and claims, it does not exclude other elements or steps. For the purpose of the present invention, the term "consisting of" is considered to be an optional embodiment of the term "comprising". If hereinafter a group is defined to comprise at least a certain number of embodiments, this is also to be understood to disclose a group which optionally consists only of these embodiments.

Where an indefinite or a definite article is used when referring to a singular noun e.g. "a" or "an", "the", this includes a plural form of that noun unless specifically stated.

*Vice versa*, when the plural form of a noun is used it refers also to the singular form.

Furthermore, the terms first, second, third or (a), (b), (c) and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein unless context clearly indicates otherwise.

In the context of the present invention any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%. The aforementioned deviation from the indicated numerical interval of ± 10%, and preferably of ± 5% is also indicated by the terms "about" and "approximately" used herein with respect to a numerical value.

According to the first aspect of the present invention, a method for detecting the presence of a dengue virus serotype in a sample containing at least one dengue virus serotype comprising dengue virus nucleic acids is provided comprising the steps:
a) optionally purifying the dengue virus nucleic acids from said sample;
b) preparing double-stranded DNA templates from said dengue virus nucleic acids;
c) amplifying said double-stranded DNA templates of step b) by polymerase chain reaction (PCR) using a DNA polymerase with a primer pair selected from the group consisting of
   (i) a dengue serotype 1 (DENV-1) specific primer pair, wherein the forward primer comprises the nucleotide sequence set forth in SEQ ID NO: 1 or a variant thereof having at least 85% sequence identity thereof and the reverse primer comprises the nucleotide sequence set forth in SEQ ID NO: 2 or a variant thereof having at least 85% sequence identity thereof such that in the presence of the DENV-1 serotype an amplicon having a length of about 400 bp to about 500 bp generated;
   (ii) a dengue serotype 2 (DENV-2) specific pair, wherein the forward primer comprises the nucleotide sequence set forth in SEQ ID NO: 3 or a variant thereof having at least 85% sequence identity thereof and the reverse primer comprises the nucleotide sequence set forth in SEQ ID NO: 4 or a variant thereof having at least 85% sequence identity thereof such that in the presence of the DENV-2 serotype an amplicon having a length of about 300 bp to about 400 bp is generated;
   (iii) a dengue serotype 3 (DENV-3) specific primer pair, wherein the forward primer comprises the nucleotide sequence set forth in SEQ ID NO: 5 or a variant thereof having at least 85% sequence identity thereof and the reverse primer comprises the nucleotide sequence set forth in SEQ ID NO: 6 or a variant thereof having at least 85% sequence identity thereof such that in the presence of the DENV-3 serotype an amplicon having a length of about 700 bp to about 800 bp is generated; and/or
   (iv) a dengue serotype 4 (DENV-4) specific primer pair, wherein the forward primer comprises the nucleotide sequence set forth in SEQ ID NO: 7 or a variant thereof having at least 85% sequence identity thereof and the reverse primer comprises the nucleotide sequence set forth in SEQ ID NO: 8 or a variant thereof having at least 85% sequence identity thereof such that in the presence of DENV-4 serotype an amplicon having a length of about 200 bp to about 300 bp is generated;
d) separating the generated amplicon(s); and
e) detecting the generated amplicon(s), wherein the presence of an amplicon having a length of about 400 bp to about 500 bp is indicative for the presence of DENV-1, the presence of an amplicon having a length of about 300 bp to about 400 bp is indicative for the presence of DENV-2, the presence of an amplicon having a length of about 700 bp to about 800 bp is indicative for the presence of DENV-3 and/or the presence of an amplicon having a length of about 200 bp to about 300 bp is indicative for the presence of DENV-4.

In an embodiment, the inventive method consists of steps a) to e). In another embodiment, the inventive method consists of steps b) to e).

There are four known "dengue virus serotypes", dengue-1 (DENVl), dengue-2 (DENV-2), dengue-3 (DENV-3), and dengue-4 (DENV-4). Dengue virus serotypes 1-4 can also cause dengue hemorrhagic fever (DHF) and dengue shock syndrome (DSS). In the context of the invention, a virus comprising the prM-E genes of DENV-1, 2, 3, or 4, is referred to as dengue serotype 1, 2, 3, or 4, respectively, even if the backbone is of another serotype, e.g. DENV-2, or virus, e.g. yellow fever virus.

An RNA virus can be characterized by its RNA sequence. It is, alternatively or in addition, also common practice to characterize the genome of RNA viruses by the corresponding DNA sequences and corresponding DNA sequences are readily understood to reflect the RNA genome in the virus. Therefore, reference to "t" or "thymine" in the entire disclosure is to be understood as reference to "u" or "uracil", respectively, if the genomic RNA virus sequence is meant.

"Sample" herein means any sample obtained from a vaccinated individual or a Dengue virus infected patient (also referred to herein as a "Dengue disease patient"), or any sample to be tested at the stage of quality control of manufactured vaccines, i.e. a sample containing one to four live, e.g. one, two, three, or four attenuated Dengue virus vaccine strains. If the sample is from a vaccinated individual or Dengue disease patient, it may be from whole blood or serum, most preferably from serum. If the sample is to be tested at the stage of quality control, it may also be a vaccine composition or a stock solution of a vaccine composition prior to administration. In such a scenario, testing according to the inventive method may be important for quality control and safety reasons.

In a preferred embodiment, the inventive method is performed on a sample from a dengue disease patient.

"An attenuated virus" herein means that the virus has a reduced replication capacity and/or a reduced infectivity in host cells compared to wild-type virus. The replication capacity and/or infectivity may be determined *in vitro* in suitable cell systems or *in vivo* in suitable animal models. Attenuation may be achieved by serial passaging of the virus in a foreign host such as in tissue culture, embryonated eggs or live animals. Alternatively, attenuation may be performed by chemical agents.

"Live attenuated" viruses are important for use in viral vaccines, since the live attenuated virus generates a stronger immune response compared to an inactivated virus. The live attenuated virus may be an RNA virus or a DNA virus. Suitable live attenuated RNA viruses may be selected from dengue virus, poliovirus, rubella virus, measles virus, yellow fever virus, mumps virus, zika virus, and influenza virus. Preferably, the live attenuated RNA virus is dengue virus. Suitable live attenuated DNA viruses include varicella zoster virus, vaccinia virus, smallpox virus, Herpes simplex virus and chikungunya virus. The live attenuated virus may differ from the wild-type virus in one or more mutations in the nucleic acid sequence of the virus. For example, for one tetravalent dengue vaccine (TDV) it is known that attenuation of neurovirulence in newborn mice is determined by mutations 5'NCR-57 C->U, NS1-53 Gly- >Asp (nt-2579 G->A) and NS3-250 Glu->Val (nt-5270 A->U) (Butrapet et al., J. Virol. 74 (2000), 3011-3019). The above nucleotide sequence numbering relates to the nucleotide sequence of the live attenuated Dengue virus serotype 2 (TDV-2) set forth in SEQ ID NO:10.

A "vaccine composition" or "vaccine formulation" as used herein means a composition containing antigens of one to four, e.g. one, two, three, or four, of the dengue serotypes and formulated for administration to an individual. Preferably, a vaccine composition comprises a dengue antigen of each of serotypes 1 to 4 which are each independently selected from the group consisting of: (a) a live attenuated dengue virus and (b) a live attenuated chimeric virus.

In a preferred embodiment, a vaccine composition comprises a dengue antigen of each of serotypes 1 to 4, wherein said dengue antigens of serotypes 1, 3 and 4 are each a live attenuated chimeric dengue virus and said dengue antigen of serotype 2 is selected from the group consisting of a live attenuated dengue virus and a live attenuated chimeric dengue virus. For example, a vaccine composition may comprise a dengue antigen of each of serotypes 1 to 4, wherein said dengue antigens of serotypes 1, 3 and 4 are each a live attenuated chimeric dengue/dengue virus and said dengue antigen of serotype 2 is a live attenuated dengue virus. For example, a vaccine composition may be the tetravalent mixture of dengue antigens of each of serotypes 1 to 4 (referred to as DENVax) which is disclosed in Huang et al., PLoS Negl Trop Dis 7(5): e2243 (2013). The four serotypes, TDV-1, TDV-2, TDV-3, and TDV-4, have the sequences of SEQ ID NOs: 9-12, respectively.

In another preferred embodiment, a vaccine composition may comprise a dengue antigen of each of serotypes 1 to 4, wherein said dengue antigens of serotypes 1, 3 and 4 are each a live attenuated chimeric yellow fever (YF)/dengue virus and said dengue antigen of serotype 2 is a live attenuated dengue virus.

In another preferred embodiment, a vaccine composition comprises a dengue antigen of each of serotypes 1 to 4, wherein said dengue antigens of serotypes 1 , 3 and 4 are each independently selected from the group consisting of: (a) a live attenuated dengue virus and (b) a live attenuated chimeric dengue virus and said dengue antigen of serotype 2 is a live attenuated chimeric dengue virus. For example, a vaccine composition may comprise a dengue antigen of each of serotypes 1 to 4, wherein said dengue antigens of serotypes 1, 3 and 4 are each a live attenuated dengue virus and said dengue antigen of serotype 2 is a live attenuated chimeric dengue/dengue virus. For example, a vaccine composition may be any of the tetravalent mixtures (referred to as TV001 , TV002, TV003 and TV004') of dengue antigens of each of serotypes 1 to 4 which are disclosed in Durbin et al., Journal of Infectious Diseases (2013), 207, 957-965. Preferably, a vaccine composition is TV003.

In another preferred embodiment, a vaccine composition comprises a dengue antigen of each of serotypes 1 to 4, wherein each of said dengue antigens is a live attenuated chimeric dengue virus, preferably a chimeric YF/dengue virus, more preferably a chimeric YF/dengue virus which comprises an attenuated YF genomic backbone whose prM-E sequence has been substituted with the prM-E sequence of dengue virus.

In a preferred embodiment, a live attenuated chimeric dengue virus comprises one or more proteins from a dengue virus and one or more proteins from a different flavivirus. Preferably, the different flavivirus is a yellow fever virus (i.e. a chimeric YF/dengue virus). Preferably a live attenuated chimeric dengue virus comprises an attenuated yellow fever virus genome whose prM-E sequence has been substituted with the prM-E sequence of a dengue virus. Alternatively, a live attenuated chimeric dengue virus comprises one or more proteins from a first dengue virus and one or more proteins from a second dengue virus (i.e. a chimeric dengue/dengue virus). Preferably said first dengue virus and said second dengue virus are of different serotypes. Where said first dengue virus and said second dengue virus are of the same serotype, said first and second dengue viruses are different strains of the same serotype.

Accordingly, in a preferred embodiment of any of the above methods, the inventive method is performed on a sample comprising at least two different dengue serotypes, preferably at least three different dengue serotypes, and more preferably each of the four different serotypes. In an embodiment, the sample contains only two different dengue serotypes, only three dengue serotypes, or only all four different dengue serotypes.

In an especially preferred embodiment, the inventive method is performed on a sample that is a vaccine formulation and comprises at least one live, attenuated dengue virus, preferably at least two different live, attenuated dengue viruses, more preferably at least three different live, attenuated dengue viruses, and most preferably at least four different live, attenuated viruses. In one such embodiment, the at least one live, attenuated virus comprises one or more of a nucleotide sequence selected from the group consisting of SEQ ID NOs: 9 to 12 (TDV-1 to TDV-4), or a variant of the foregoing having at least 90% sequence identity. That is, in an embodiment, the inventive method is performed on a sample that is a vaccine formulation and comprises or consists of TDV-1, TDV-2, TDV-3, or TDV-4 or a variant of the foregoing having at least 90% sequence identity. In another embodiment, the inventive method is performed on a sample that is a vaccine formulation and comprises or consists of TDV-1 and TDV-2 or (a) variant(s) of the foregoing having at least 90% sequence identity; TDV-1 and TDV-3 or (a) variant(s) of the foregoing having at least 90% sequence identity; TDV-1 and TDV-4 or (a) variant(s) of the foregoing having at least 90% sequence identity; TDV-2 and TDV-3 or (a) variant(s) of the foregoing having at least 90% sequence identity; TDV-2 and TDV-4 or (a) variant(s) of the foregoing having at least 90% sequence identity; or TDV-3 and TDV-4 or (a) variant(s) of the foregoing having at least 90% sequence identity. In another embodiment, the inventive method is performed on a sample that is a vaccine formulation and comprises or consists of TDV-1, TDV-2, and TDV-3 or (a) variant(s) of the foregoing having at least 90% sequence identity; TDV-1, TDV-2 and TDV-4; TDV-1, TDV-3, and TDV-4 or (a) variant(s) of the foregoing having at least 90% sequence identity; or TDV-2, TDV-3, and TDV-4 or (a) variant(s) of the foregoing having at least 90% sequence identity. In another embodiment, the inventive method is performed on a sample that is a vaccine formulation and comprises or consists of all of TDV-1, TDV-2, TDV-3, and TDV-4 or (a) variant(s) of the foregoing having at least 90% sequence identity. Preferably, in any of the above embodiments, the sequence identity of the variant(s) is at least 90, 91, 92, 93, 94, 9, 96, 97, 98, or 99.5%.

"Purifying virus nucleic acids from the sample" may be performed using any method for DNA or RNA isolation known in the art. Suitable methods comprise *inter alia* steps such as centrifugation steps, precipitation steps, chromatography steps, dialyzing steps, heating steps, cooling steps and/or denaturation steps. Any commercially available kit for the purification of nucleic acids may be used. Where the dengue nucleic acids are RNAs, any known method and/or any commercially available kit for RNA purification may be used.

"Preparing double-stranded DNA templates" herein means that if the virus nucleic acid is an RNA, step b) includes a step of reverse transcription with a reverse transcriptase, a reverse primer and a mixture of nucleotides such that a RNA/DNA hybrid is generated which is subsequently converted into a double-stranded cDNA template. The reaction may be carried out in an automated thermal cycler. The reaction may be a one-step RT-PCR also encompassing step c) of the inventive method. Any method known in the art and any commercially available, reverse transcriptase, kit and/or thermal cycler may be used.

Accordingly, in a preferred embodiment of any of the above methods, the virus nucleic acids are isolated virus RNA and step b) comprises a step of reverse transcription of the isolated virus RNA in the presence of a reverse transcriptase using a reverse primer.

If the virus nucleic acid is a single-stranded DNA, the double-stranded DNA-template is generated by the activity of a template-dependent DNA polymerase. Any method known in the art and any commercially available polymerase, kit and/or thermal cycler may be used.

"Amplifying said double-stranded templates from step b)" herein means that the double-stranded DNA templates of step b) are incubated with the inventive primer pair(s) (forward and reverse primers), nucleotides and a template-dependent DNA polymerase such that a PCR product is generated for each instance of a template of one serotype and matching primer pair for that same serotype, wherein the PCR product comprises parts of the E gene of said dengue virus serotype. The reaction may be carried out in an automated thermal cycler. Any PCR method known in the art and any commercially available polymerase, kit, and/or thermal cycler may be used. In a preferred embodiment of any of the above methods, the reaction may be a one-step RT-PCR also encompassing step b) of the inventive method. Preferably, the PCR amplification reaction of step c) comprises at least 10 PCR cycles using a DNA polymerase, the inventive primer pairs, and nucleotides. More preferably, 10 to 50 cycles, even more preferred 20 to 45 cycles are used. Most preferably, 35 cycles are used. It is also preferred that the PCR amplification reaction includes a denaturing step at a temperature from about 90°C to about 99°C, an annealing step at a temperature from about 45°C to about 75°C, and an extension step at a temperature from about 60°C to about 75°C, more preferably denaturing is performed at about 95°C, annealing is performed at 66°C and extension is performed at 72°C. In a preferred embodiment, the amplifying step c) is carried out in a thermocycling process at +50°C for 20 min; +95°C for 2 min; and multiple cycles of +95°C for 30 s; +66°C for 30s and +72°C for 1 min. In a most preferred such embodiment, the multiple cycles are 35 cycles.

The term "primer" as used herein denotes an oligonucleotide that acts as an initiation point of nucleotide synthesis under conditions in which synthesis of a primer extension product complementary to a nucleic acid strand is induced. The primers may be generated such that they are able to bind to the sequence to be reverse transcribed or amplified. The primer may comprise from 5 to 50 nucleotides, preferably, 8 to 30 nucleotides; more preferably the primer comprises 15 to 25 nucleotides. The primer sequences are complementary to at least a portion of the sequence to be reverse transcribed or amplified. Preferably, the primer sequences are fully complementary to a sequence within the sequence to be reverse transcribed or amplified. Methods for the design of sequence specific primers and probes are known in the art.

The inventive primer pairs are specific for one of the four dengue serotypes each. More specifically, they are specific for the E gene of one of the four dengue serotypes each, such that each inventive primer pair will generate a PCR product, also referred to as "amplicon", that covers part of the respective E gene only when a double-stranded DNA template comprising the E gene of the matching serotype is present in the amplification reaction of step c). No amplicon will be generated when only double-stranded DNA templates comprising the E gene of the other three serotypes are present. That is, each of the inventive primer pairs will produce amplicons for only the matching DENV type or a chimeric virus comprising the E gene from the matching DENV type, e.g. the matching TDV type.

The inventive primer pairs are as follows:
(i) a dengue serotype 1 (DENV-1) specific primer pair, wherein the forward primer comprises or consists of the nucleotide sequence set forth in SEQ ID NO: 1 or a variant thereof having at least 85% sequence identity thereof and the reverse primer comprises or consists of the nucleotide sequence set forth in SEQ ID NO: 2 or a variant thereof having at least 85% sequence identity thereof;
(ii) a dengue serotype 2 (DENV-2) specific pair, wherein the forward primer comprises or consists of the nucleotide sequence set forth in SEQ ID NO: 3 or a variant thereof having at least 85% sequence identity thereof and the reverse primer comprises or consists of the nucleotide sequence set forth in SEQ ID NO: 4 or a variant thereof having at least 85% sequence identity thereof;
(iii) a dengue serotype 3 (DENV-3) specific primer pair, wherein the forward primer comprises or consists of the nucleotide sequence set forth in SEQ ID NO: 5 or a variant thereof having at least 85% sequence identity thereof and the reverse primer comprises or consists of the nucleotide sequence set forth in SEQ ID NO: 6 or a variant thereof having at least 85% sequence identity thereof; and
(iv) a dengue serotype 4 (DENV-4) specific primer pair, wherein the forward primer comprises or consists of the nucleotide sequence set forth in SEQ ID NO: 7 or a variant thereof having at least 85% sequence identity thereof and the reverse primer comprises or consists of the nucleotide sequence set forth in SEQ ID NO: 8 or a variant thereof having at least 85% sequence identity thereof.

The forward primer of the inventive primer pair specific for dengue serotype 1 may have 85, 86, 87, 88, 89, 90, 91, 92, 93, 94, 95, 96, 97, 98, 99, 99.5, or 100% sequence identity to SEQ ID NO: 1. In an embodiment, the forward primer with at least 85 sequence identity has the same length as SEQ ID NO: 1.

The reverse primer of the inventive primer pair specific for dengue serotype 1 may have 85, 86, 87, 88, 89, 90, 91, 92, 93, 94, 95, 96, 97, 98, 99, 99.5, or 100% sequence identity to SEQ ID NO: 2. In an embodiment, the reverse primer with at least 85 sequence identity has the same length as SEQ ID NO:2.

The forward primer of the inventive primer pair specific for dengue serotype 2 may have 85, 86, 87, 88, 89, 90, 91, 92, 93, 94, 95, 96, 97, 98, 99, 99.5, or 100% sequence identity to SEQ ID NO: 3. In an embodiment, the forward primer with at least 85 sequence identity has the same length as SEQ ID NO:3.

The reverse primer of the inventive primer pair specific for dengue serotype 2 may have 85, 86, 87, 88, 89, 90, 91, 92, 93, 94, 95, 96, 97, 98, 99, 99.5, or 100% sequence identity to SEQ ID NO: 4. In an embodiment, the reverse primer with at least 85 sequence identity has the same length as SEQ ID NO:4.

The forward primer of the inventive primer pair specific for dengue serotype 3 may have 85, 86, 87, 88, 89, 90, 91, 92, 93, 94, 95, 96, 97, 98, 99, 99.5, or 100% sequence identity to SEQ ID NO: 5. In an embodiment, the forward primer with at least 85 sequence identity has the same length as SEQ ID NO:5.

The reverse primer of the inventive primer pair specific for dengue serotype 3 may have 85, 86, 87, 88, 89, 90, 91, 92, 93, 94, 95, 96, 97, 98, 99, 99.5, or 100% sequence identity to SEQ ID NO: 6. In an embodiment, the reverse primer with at least 85 sequence identity has the same length as SEQ ID NO:6.

The forward primer of the inventive primer pair specific for dengue serotype 4 may have 85, 86, 87, 88, 89, 90, 91, 92, 93, 94, 95, 96, 97, 98, 99, 99.5, or 100% sequence identity to SEQ ID NO: 7. In an embodiment, the forward primer with at least 85 sequence identity has the same length as SEQ ID NO:7.

The reverse primer of the inventive primer pair specific for dengue serotype 4 may have 85, 86, 87, 88, 89, 90, 91, 92, 93, 94, 95, 96, 97, 98, 99, 99.5, or 100% sequence identity to SEQ ID NO: 8. In an embodiment, the reverse primer with at least 85 sequence identity has the same length as SEQ ID NO:8.

The inventive primer pair specific for dengue serotype 1 (DENV-1) anneals to sequences of the E gene of dengue serotype 1, and thus generates an amplicon that covers part of the E gene only when a double-stranded DNA template comprising the E gene of the serotype 1, e.g. DENV-1 or TDV-1, is present in the amplification reaction of step c). No amplicon will be generated when only double-stranded DNA templates comprising the E gene of the other serotypes 2-4, e.g. DENV-2, DENV-3, DENV-4, TDV-2, TDV-3, and/or TDV-4, are present. The amplicon generated by this inventive primer pair is about 400bp to about 500bp in length. More preferably, when the forward and reverse primer have 100% sequence identity to SEQ ID NOs: 1 and 2, respectively, the amplicon has a length of 419 bp.

The inventive primer pair specific for dengue serotype 2 (DENV-2) anneals to sequences of the E gene of dengue serotype 2, and thus generates an amplicon that covers part of the E gene only when a double-stranded DNA template comprising the E gene of the serotype 2, e.g. DENV-2 or TDV-2, is present in the amplification reaction of step c). No amplicon will be generated when only double-stranded DNA templates comprising the E gene of the other serotypes 1 and 3-4, e.g. DENV-1, DENV-3, DENV-4, TDV-1, TDV-3, and/or TDV-4, are present. The amplicon generated by this inventive primer pair is about 300bp to about 400bp in length. More preferably, when the forward and reverse primer have 100% sequence identity to SEQ ID NOs: 3 and 4, respectively, the amplicon has a length of 390 bp.

The inventive primer pair specific for dengue serotype 3 (DENV-3) anneals to sequences of the E gene of dengue serotype 3, and thus generates an amplicon that covers part of the E gene only when a double-stranded DNA template comprising the E gene of the serotype 3, e.g. DENV-3 or TDV-3, is present in the amplification reaction of step c). No amplicon will be generated when only double-stranded DNA templates comprising the E gene of the other serotypes 1-2 and 4, e.g. DENV-1, DENV-2, DENV-4, TDV-1, TDV-2, and/or TDV-4, are present. The amplicon generated by this inventive primer pair is about 700bp to about 800bp in length. More preferably, when the forward and reverse primer have 100% sequence identity to SEQ ID NOs: 5 and 6, respectively, the amplicon has a length of 781 bp.

The inventive primer pair specific for dengue serotype 4 (DENV-4) anneals to sequences of the E gene of dengue serotype 4, and thus generates an amplicon that covers part of the E gene only when a double-stranded DNA template comprising the E gene of the serotype 4, e.g. DENV-4 or TDV-4, is present in the amplification reaction of step c). No amplicon will be generated when only double-stranded DNA templates comprising the E gene of the other serotypes 1-3, e.g. DENV-1, DENV-2, DENV-3, TDV-1, TDV-2, and/or TDV-3, are present. The amplicon generated by this inventive primer pair is about 200bp to about 300bp in length. More preferably, when the forward and reverse primer have 100% sequence identity to SEQ ID NOs: 7 and 8, respectively, the amplicon has a length of 226 bp.

Accordingly, in any of the methods disclosed herein, the amplicon generated by the DENV-1 specific primer pair may have a length of about 419 bp, the amplicon generated by the DENV-2 specific primer pair may have a length of about 390 bp, the amplicon generated by the DENV-3 specific primer pair may have a length of about 731 bp, and the amplicon generated by the DENV-4 specific primer may have a length of about 226 bp.

"Separating the generated amplicon(s)" herein means that the PCR product(s) obtained in step c) are separated by size. Any of the well-known methods in the art for separating DNA molecules by size may be used. In a preferred embodiment of any of the above methods, step d) thus includes gel electrophoresis. Gel electrophoresis is a laboratory method used to separate mixtures of DNA according to molecular size. In gel electrophoresis, the molecules to be separated are pushed by an electrical field through a gel that contains small pores. The molecules travel through the pores in the gel at a speed that is inversely related to their lengths. This means that a small DNA molecule will travel a greater distance through the gel than will a larger DNA molecule. The differently sized molecules will thus migrate in bands. The electrical field is applied such that one end of the gel has a positive charge and the other end has a negative charge. Because DNA consist of negatively charged molecules, these molecules will be pulled toward the positively charged end of the gel. Finally, after the DNA molecules have been separated using gel electrophoresis, bands representing molecules of different sizes can be detected. Preferably, the gel used for gel electrophoresis is an agarose gel. The percentage of agarose in the agarose gel preferably is 0.5-3%, more preferably 1-2%, most preferably 2%. In another preferred embodiment, the PCR product(s) obtained in step c) are separated by capillary electrophoresis. Capillary electrophoresis is well-known to the skilled person. Preferably, the capillary electrophoresis is a capillary gel electrophoresis, wherein the separation of the nucleic acids is performed in a capillary filled with a gel containing a dye. During the electric field-forced migration of the nucleic acids through the gel the dye attaches to the nucleic acids and is determined by a detector. Commercially available electrophoresis devices and cartridges include the QIAxcel^{®} Advanced System and cartridges from Qiagen.

"Detecting the generated amplicon(s)" herein means that the bands in which the amplicon(s) travel(s) on the gel of step d) is/are visualized. Any of the well-known methods known in the art for visualizing DNA molecules may be used. Visualization typically involves staining with dyes with low intrinsic fluorescence, a strong affinity for DNA, and a high quantum yield of fluorescence after binding to nucleic acids. The greater the increase in quantum yield, the higher the ratio of signal to noise. Bands of DNA stained with these dyes are visualized by illuminating the gel with UV light at one wavelength and recording at another. Preferably, the fluorescent dye is ethidium bromide. Accordingly, in a preferred embodiment of any of the above methods, step e) comprises visualization of the amplicon(s) by staining, and preferably ethidium bromide staining is used. The staining can also be performed in the above separation step. For example, the capillary electrophoresis gel used for the separation may also include the dye or stain. Upon application of the sample to the gel the double-stranded PCR product(s) adsorb the dye or stain which can then be used for the visualization.

In a preferred embodiment of any of the above methods, the inventive method further comprises sequencing of the generated amplicon(s). In an embodiment, the inventive method consists of steps a) to e) and the sequencing step. In another embodiment, the inventive method consists of steps b) to e) and the sequencing step. Any method known in the art for sequencing PCR products may be used. For example, Sanger sequencing can be used. Sanger sequencing is based on the detection of labelled chain-terminating nucleotides that are incorporated by a DNA polymerase during the replication of a template (here: isolated amplicons). Typically, bands separated and detected as in step d) may be excised from the gel, and the isolated molecules contained therein can be purified by well-known methods in the art and commercially available kits. The resulting purified isolated DNA corresponding to the amplicons generated in step c) can then be used directly for sequencing.

In an embodiment of any of the above inventive methods, the sample comprises residual host cell DNA. While host cell DNA may be removed during optional step a), this is not necessary for the inventive method. The specificity of the inventive primer pairs is such that no unspecific signal will be generated in step c) even in the presence of host cell DNA.

"Host cell" as used herein can mean cells from a vaccinated individual or Dengue virus infected patient found in a sample obtained from the individual or patient. Alternatively, "host cell" as used herein may refer to cells used for the production of vaccine strains, preferably live, attenuated dengue virus strains, more preferably TDV-1 to TDV-4. Suitable host cells for vaccine strain production include mammalian cells such as, e.g., Vero cells. Accordingly, in a preferred embodiment of any of the above methods, prior to step a), the live, attenuated dengue virus is grown in Vero cells. It is preferred that Vero cells are seeded in T25 cm² flasks at 1.5×10⁶ cells/flask and grown overnight to 80-90% confluency by incubating at 37°C with 5% CO₂ tension. Preferably, the virus is diluted in assay medium DMEM containing 1% FBS and 1% Penicillin/Streptomycin. Preferably, Vero cells are infected with TDV strains (e.g. TDV-1 to TDV-4) at 0.1MOI and allowed virus adsorption for 1 hour at 37°C 5% CO₂ incubator.

According to a second aspect, the present invention provides the use of any of the inventive methods according to the first aspect of the invention in the quality control of vaccines containing live, attenuated dengue virus. For the reliable determination of the serotypes of both the monovalent drug substance comprising a single live, attenuated dengue virus and the tetravalent drug product comprising a mixture of all four live, attenuated viruses it is essential to confirm the presence of (only) the dengue virus serotype(s) in the vaccine formulation before batches are released for administration to individuals.

According to a third aspect, the present invention provides the use of any of the inventive methods according to the first aspect of the invention in the diagnosis of a virus infection in a patient sample. The method according to the first aspect may be used for the diagnosis of a virus infection in a virus infected disease patient. In particular, the presence of one or more amplicons in step e) of the inventive method is indicative of infection of the patient with dengue virus. The method also enables the detection of co-infection with different subtypes of viruses such as different dengue serotypes. This may provide advantages in the personalized treatment of the infected patients. Preferably, the method for the diagnosis of a virus infection in a virus infected patient is carried out *in vitro* on a biological sample previously obtained from the patient. Preferably, the sample is a blood sample, more preferably the sample is a serum sample. Alternatively, the method according to the first aspect may be used for the diagnosis of a virus infection in a recently vaccinated individual to confirm successful infection with live, attenuated viruses. In particularly, if the vaccine administered to the individual was multivalent, e.g. tetravalent, successful infection with all included serotypes can be confirmed.

According to a further aspect of the present invention a kit is provided for detecting the presence of dengue virus serotype in a sample containing at least one dengue virus comprising:
(i) a dengue serotype 1 (DENV-1) specific primer pair, wherein the forward primer comprises the nucleotide sequence set forth in SEQ ID NO: 1 or a variant thereof having at least 85% sequence identity thereof and the reverse primer comprises the nucleotide sequence set forth in SEQ ID NO: 2 or a variant thereof having at least 85% sequence identity thereof;
(ii) a dengue serotype 2 (DENV-2) specific pair, wherein the forward primer comprises the nucleotide sequence set forth in SEQ ID NO: 3 or a variant thereof having at least 85% sequence identity thereof and the reverse primer comprises the nucleotide sequence set forth in SEQ ID NO: 4 or a variant thereof having at least 85% sequence identity thereof;
(iii) a dengue serotype 3 (DENV-3) specific primer pair, wherein the forward primer comprises the nucleotide sequence set forth in SEQ ID NO: 5 or a variant thereof having at least 85% sequence identity thereof and the reverse primer comprises the nucleotide sequence set forth in SEQ ID NO: 6 or a variant thereof having at least 85% sequence identity thereof; and/or
(iv) a dengue serotype 4 (DENV-4) specific primer pair, wherein the forward primer comprises the nucleotide sequence set forth in SEQ ID NO: 7 or a variant thereof having at least 85% sequence identity thereof and the reverse primer comprises the nucleotide sequence set forth in SEQ ID NO: 8 or a variant thereof having at least 85% sequence identity thereof.

These primers are as described above for the first aspect of the invention.

In an embodiment, the kit consists of one, two, three, or all four of the specific primer pairs. That is, in an embodiment, the kit comprises or consists of the primer pair specific for DENV-1, DENV-2, DENV-3, or DENV-4. In an embodiment, the kit comprises or consists of the primer pair specific for DENV-1 and DENV-2; DENV-1 and DENV-3; DENV-1 and DENV-4; DENV-2 and DENV-3; DENV-2 and DENV-4; or DENV-3 and DENV-4. In an embodiment, the kit comprises or consists of the primer pairs for DENV-1, DENV-2, and DENV-3; DENV-1, DENV-2 and DENV-4; DENV-1, DENV-3 and DENV-4; or DENV-2, DENV-3, and DENV-4. In an embodiment, the kit consists of or comprises the primer pairs for DENV-1, DENV-2, DENV-3, and DENV-4.

In another embodiment, the inventive kit may further include a suitable reverse transcriptase, nucleotides and suitable buffers known in the art, as described above for step b) of the inventive method of the first aspect of the invention.

According to a fourth aspect, the present invention provides the use of any of the inventive kits according to the third aspect of the invention in the quality control of vaccines containing live, attenuated dengue virus. The benefits of this aspect are as described above for the second aspect of the invention.

According to a fifth aspect, the present invention provides the use of any of the inventive kits according to the third aspect of the invention in the diagnosis of a virus infection in a patient sample. The benefits of this aspect are as described above for the third aspect of the invention.

According to a sixth aspect of the present invention, a quality control method for vaccines containing live, attenuated dengue virus is provided comprising or consisting of performing the method of the first aspect of the invention and at least one further method selected from the group consisting of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s).

In an embodiment, the quality control method comprises or consists of the method of the first aspect of the invention and at least two further method selected from the group consisting of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least three further method selected from the group consisting of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least four further method selected from the group consisting of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least five further method selected from the group consisting of immunofocus assay, attenuation check, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least six further method selected from the group consisting of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least seven further method selected from the group consisting of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least eight further method selected from the group consisting of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and at least nine further method selected from the group consisting of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s). In an embodiment, the quality control method comprises or consists of the method of the first aspect of the invention and all of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s).

In a preferred embodiment the quality control method comprises performing the method according to the present invention and an attenuation check method comprising the steps of:
a) purifying flavivirus nucleic acids from said formulation;
b) preparing double-stranded DNA templates from said flavivirus nucleic acids;
c) amplifying said viral nucleic acids of a) or said double-stranded DNA templates of b) by polymerase chain reaction (PCR) by using a primer pair comprising sequences specific for a flavivirus sequence comprising the at least one attenuation locus such that PCR products comprising the at least one attenuation locus are generated;
d) sequencing said PCR products by next generation sequencing by synthesis selected from HiSeq sequencing, MiSeq sequencing and pyrosequencing; and
e) determining the nucleotide sequence at the at least one attenuation locus.

MiSeq sequencing and HiSeq sequencing technologies are commercially available from Illumina Inc. pyrosequencing may be carried out using the commercial Roche 454 sequencing platform. Preferably, MiSeq sequencing is used.

In a particularly preferred embodiment the live, attenuated flavivirus is a dengue virus comprising three attenuation loci at positions 57, 2579 and 5270 of the nucleotide sequence of dengue virus type 2 according to SEQ ID NO: 10 and the primer pair of step c) is selected from the group consisting of:
i) a forward primer comprising SEQ ID NO: 17, or a variant thereof having at least 85% sequence identity thereof and a reverse primer comprising SEQ ID NO: 18, or a variant thereof having at least 85% sequence identity thereof such that a first PCR product having a length of about 310 bp to about 330 bp and comprising the first attenuation locus is generated;
ii) a forward primer comprising SEQ ID NO: 19, or a variant thereof having at least 85% sequence identity thereof and a reverse primer pair comprising SEQ ID NO: 20, or a variant thereof having at least 85% sequence identity thereof such that a second PCR product having a length of about 320 bp to about 350 bp and comprising the second attenuation locus is generated; and
iii) a forward primer comprising SEQ ID NO: 21, or a variant thereof having at least 85% sequence identity thereof and a reverse primer comprising SEQ ID NO: 22, or a variant thereof having at least 85% sequence identity thereof such that a third PCR product having a length of about 400 bp to about 430 bp and comprising the third attenuation locus is generated.

In another preferred embodiment the quality control method comprises performing the method according to the present invention and quantitative determination of human serum albumin (HSA) monomer.

In a particularly, preferred embodiment the method for quantitative determination of human serum albumin (HSA) monomer comprises the steps of:
(a) applying about 5 µL to about 15 µL of the liquid composition on a size-exclusion chromatography column, wherein the column material comprises diol residues coupled to a silica gel;
(b) performing size-exclusion chromatography at a flow rate of about 0.7 to about 0.9 mL/min with a mobile phase comprising about 0.08 to about 0.12 M phosphate buffer with about 0.08 M to about 0.12 M sodium sulfate at a pH value from about 6.8 to about 7.0;
(c) detecting the UV absorption of the HSA monomer at the outlet of the column; and
(d) calculating the amount of HSA monomer from the area under the curve (AUC) of the detected signal.

In one embodiment, the one or more excipients may be selected from the group consisting of trehalose, poloxamer, urea, arginine hydrochloride, tromethamine, human serum albumin, chloride salts, and phosphate salts or any combination thereof. Preferably, trehalose is α,α-trehalose dihydrate. Preferably, poloxmer is poloxamer 407, also known by its trade names Pluronic F127 and Synperonic PE/F127. Preferably, the chloride salts may comprise or consist of one or both of potassium chloride and sodium chloride. Preferably, the phosphate salts may comprise or consist of one or both of potassium dihydrogen phosphate and disodium hydrogen phosphate.

In one preferred such embodiment, the excipients are a combination of trehalose, at least one poloxamer, and human serum albumin. More preferably, the excipients are a combination of trehalose, poloxamer, human serum albumin, chloride salts, and phosphate salts. Most preferably, the excipients are a combination of α,α-trehalose dihydrate, poloxamer 407, human serum albumin, potassium chloride, sodium chloride, potassium dihydrogen phosphate, and disodium hydrogen phosphate.

In another preferred such embodiment, the excipients are a combination of trehalose, at least one poloxamer, urea, arginine hydrochloride, tromethamine and human serum albumin. More preferably, the excipients are a combination of trehalose, poloxamer, urea, arginine hydrochloride, tromethamine, human serum albumin, chloride salts and phosphate salts. Preferably, the chloride salts comprise or consist of sodium chloride.

The invention is further described in the following examples which are solely for the purpose of illustrating specific embodiments of the invention, and are also not to be construed as limiting the scope of the invention in any way.

### Examples

### Sequence Identity Assay Protocol

Reverse transcription polymerase chain reaction (RT-PCR) is a sensitive method for the detection of RNA, which involves two step reactions. RNA is first reverse transcribed to cDNA by reverse transcriptase. The cDNA is then used as template for the subsequent PCR step. Amplified DNA fragments are separated and visualized in 2% agarose gel; method conditions are listed in Table 1.b.

The sequence identity of each monovalent drug substance is determined by a positive result for RT-PCR amplification of an E gene sequence specific to the appropriate dengue serotype, and a negative result for RT-PCR amplification of the E gene sequences specific to the other three dengue serotypes. Four primer sets are designed to differentiate the four serotypes of the dengue vaccine viruses. The serotype-specific primer sets were designed such that each primer has only one specific binding site within the genome of the corresponding dengue serotype and each primer set generates a DNA fragment of specific size. Viral RNA is isolated from the test sample using the QIAamp^{®} cador^{®} Pathogen Kit (corresponding to the IndiSpin Pathogen Kit (INDICAL Bioscience) according to the manufacturer's instructions.

RT-PCR on the isolated viral RNA is performed using a one-step RT-PCR kit (highQu GmbH) with the TDV-specific primer sequences identified in Table 1.a below. The amplicons for the four vaccine virus strains are 419, 390, 731 and 226 base pairs long for TDV-1, TDV-2, TDV-3 and TDV-4, respectively, and are easily differentiated on a 2% agarose gel (Table 1.a).

**Table 1.a Primer Sequences and Size of Amplicons for RT-PCR-based Sequence Identity Test**

| **Virus Strain** | | **Serotype Specific E Gene Primer Sequences** | **Amplicon Size (in Basepairs)** |
|---|---|---|---|
| TDV-1 | Forward | 5'-CCGACTACGGAACCCTTACA-3' | 419 |
| | Reverse | 5'-GGTCTCAGCCACTTCTTTCTCTA-3' | |
| TDV-2 | Forward | 5'-GCCATGCACACAGCACTTAC-3' | 390 |
| | Reverse | 5'-CTTCAGTTGTCCCGGCTCTA-3' | |
| TDV-3 | Forward | 5'-AAGCCCACGCTGGACATAG-3' | 731 |
| | Reverse | 5'-AAGTGCCCCGCAAAGATACT-3' | |
| TDV-4 | Forward | 5'-GCCGGAGAGATGTGGTAGAC-3' | 226 |
| | Reverse | 5'- CTGGGAGTTATCGTGGCTGTA -3' | |

**Table 1b: Reaction Mix components**

| **Components** | **Volume/Reaction** | | **Final Concentration** |
|---|---|---|---|
| Molecular grade water | 5.4 µL | | - |
| HighQu 1Step RT-PCR Mastermix, 2X | 10 µL | | 1X |
| HighQu RT2 Mix, 20X | 1 µL | | 1X |
| Serotype Specific Primer-pair mix (5 µM per primer) | 1.6 µL | | 0.4 µM (per primer) |
| Template | 2 µL | | - |
| Total volume | 20 µL | | - |

Accordingly, the final concentration of forward primer and reverse primer for each primer pair is 0.4 µM per primer.

**Table 1.c RT-PCR Conditions**

| **Temperature** | **Time** | **Cycles** |
|---|---|---|
| 50 °C | 20 min | 1 |
| 95 °C | 2 min | 1 |
| 95 °C | 30 sec | 35 |
| 66 °C | 30 sec | |
| 72 °C | 1 min | |
| 4 °C | 0 to infinity | 1 |

The scan of the gel along with the reference standards used for all the TDV serotypes are shown in Figure 1.a, Figure 1.b, Figure 1.c and Figure 1.d.

**Table 1.d Run conditions**

| **Run Conditions** | |
|---|---|
| Run buffer | 1xTAE |
| Concentration of agarose | 2 % |
| Voltage | 90 to 110 V |
| Course of the bromophenol blue band | 70 % |

1x TAE buffer has the following concentrations: 0.4M tris acetate 0.01M EDTA. After the run, the gel was stained at least 10 min in a 3.3 µg/ml ethidium bromide bath.

**Fig. 3** shows the analysis of samples including the tetravalent drug product by the assay according to the invention. It was observed that the assay is robust for the analysis of the presence of a given serotype in the co-presence of the three further serotypes.

### RT-PCR-based Detection of Dengue Virus Serotypes Using Different Primer Pairs (Comparative Example)

The present example differs from the above example in that instead of the inventive primer pairs the following primers were used as reverse primers in combination with the forward primers listed in Table 1:
D1-1880 (for TDV-1) (SEQ ID NO: 13); D2-1934 (for TDV-2) (SEQ ID NO: 14); D3-2046 (for TDV-3) (SEQ ID NO: 15) and D4-1997 (for TDV-4) (SEQ ID NO: 16). It was expected to obtain amplicons of 567 bp (for TDV-1), 515 bp (for TDV-2), 395 bp (for TDV-3) and 454 bp (for TDV-4).

The scan of the gel with the reference standards used for all the TDV serotypes are shown in Figure 2. The scan of the RT-PCR for TDV-1 showed only the specific band of 567 bp for TDV-1, but no fragments for the samples containing TDV-2, TDV-3 or TDV-4. However, the RT-PCR for TDV-2 resulted in the amplification of non-specific fragments having a length of > 1000 bp for the sample containing only TDV-1. Also, the RT-PCR for TDV-3 resulted in non-specific fragments of 1000 bp and about 170 bp for the sample containing TDV-1 only. The RT-PCR for TDV-4 yielded non-specific fragments of >1000 bp, about 600 bp, and about 400 bp for the sample containing only TDV-1. Further, non-specific fragments of about 700 bp and > 1000 bp, respectively, have been observed for samples containing TDV-2 and TDV-3, respectively.

From these results, it was concluded that this assay did not comply with the requirements of a specific robust sequence identity assay suitable for validation. The comparative primer pairs thus are inferior to the inventive primer pairs

### Alternative Sequence Identity Assay Protocol

The above described sequence identity assay protocol can be modified as follows:
For purifying the RNA from the dengue viruses, the IndiSpin^{®} Pathogen Kit (Indical Bioscience) may be used in accordance with manufacturer's instructions.
For separating and visualizing the generated amplicons, the QIAxcel^{®} Advanced System (Qiagen) may be used according to manufacturer's instructions. This system is based on a gel cartridge containing 12 capillaries capable of separating the applied amplicons. During the separation the amplicons are automatically stained with ethidium bromide and detected. The band size is calculated and the gel image generated.

### HUMAN SERUM ALBUMIN (SE-HPLC)

A size exclusion high-pressure liquid chromatography (SE-HPLC) method is used to identify and measure the amounts of HSA in TDV drug product. Samples are analyzed using a 30-minute isocratic method using 0.1 M phosphate buffer at pH 6.9 containing 0.1 M sodium sulfate as the mobile phase at a flow rate of 0.8 mL/minute. The reconstituted lyophilized drug product material is injected as neat sample into the YMC-Pack-Diol 200 (300 × 8 mm, 5 µm, 20 nm pore size) size exclusion column which is monitored at UV 214 nm. HSA separates into the monomer, dimer and oligomer peaks under these conditions. Accurate quantitation is achieved by comparing the test sample peak area to an external standard curve, prepared using albumin standards and assayed at the same time. The monomer concentration is reported in mg/mL.

## Claims

1. A method for detecting the presence of a dengue virus serotype in a sample containing at least one dengue virus serotype comprising dengue virus nucleic acids comprising the steps:
a) optionally purifying the dengue virus nucleic acids from said sample;
b) preparing double-stranded DNA templates from said dengue virus nucleic acids;
c) amplifying said double-stranded DNA templates of step b) by polymerase chain reaction (PCR) using a DNA polymerase with a primer pair selected from the group consisting of
(i) a dengue serotype 1 (DENV-1) specific primer pair, wherein the forward primer comprises the nucleotide sequence set forth in SEQ ID NO: 1 or a variant thereof having at least 85% sequence identity thereof and the reverse primer comprises the nucleotide sequence set forth in SEQ ID NO: 2 or a variant thereof having at least 85% sequence identity thereof such that in the presence of the DENV-1 serotype an amplicon having a length of about 400 bp to about 500 bp generated;
(ii) a dengue serotype 2 (DENV-2) specific pair, wherein the forward primer comprises the nucleotide sequence set forth in SEQ ID NO: 3 or a variant thereof having at least 85% sequence identity thereof and the reverse primer comprises the nucleotide sequence set forth in SEQ ID NO: 4 or a variant thereof having at least 85% sequence identity thereof such that in the presence of the DENV-2 serotype an amplicon having a length of about 300 bp to about 400 bp is generated;
(iii) a dengue serotype 3 (DENV-3) specific primer pair, wherein the forward primer comprises the nucleotide sequence set forth in SEQ ID NO: 5 or a variant thereof having at least 85% sequence identity thereof and the reverse primer comprises the nucleotide sequence set forth in SEQ ID NO: 6 or a variant thereof having at least 85% sequence identity thereof such that in the presence of the DENV-3 serotype an amplicon having a length of about 700 bp to about 800 bp is generated; and/or
(iv) a dengue serotype 4 (DENV-4) specific primer pair, wherein the forward primer comprises the nucleotide sequence set forth in SEQ ID NO: 7 or a variant thereof having at least 85% sequence identity thereof and the reverse primer comprises the nucleotide sequence set forth in SEQ ID NO: 8 or a variant thereof having at least 85% sequence identity thereof such that in the presence of DENV-4 serotype an amplicon having a length of about 200 bp to about 300 bp is generated;
d) separating the generated amplicon(s); and
e) detecting the generated amplicon(s), wherein the presence of an amplicon having a length of about 400 bp to about 500 bp is indicative for the presence of DENV-1, the presence of an amplicon having a length of about 300 bp to about 400 bp is indicative for the presence of DENV-2, the presence of an amplicon having a length of about 700 bp to about 800 bp is indicative for the presence of DENV-3 and/or the presence of an amplicon having a length of about 200 bp to about 300 bp is indicative for the presence of DENV-4.

2. The method of claim 1, wherein the sample comprises at least two different dengue serotypes, preferably at least three different dengue serotypes and more preferably each of the four different dengue serotypes.

3. The method of claim 1 or 2, wherein the sample is a vaccine formulation and comprises at least one live, attenuated dengue virus, preferably at least two different live, attenuated dengue viruses, more preferably at least three different live, attenuated dengue viruses and most preferably at least four different live, attenuated dengue viruses.

4. The method of claim 3, wherein the at least one live, attenuated dengue virus comprises one or more of a nucleotide sequence selected from the group consisting of SEQ ID Nos: 9 to 12 (TDV-1 to TDV-4), or a variant of the foregoing having at least 90 % sequence identity.

5. The method of any one of claims 1 to 4, wherein the sample comprises residual host cell DNA.

6. The method of any one of claims 1 to 5, wherein the amplicon generated by the DENV-1 specific primer pair has a length of about 419 bp, the amplicon generated by the DENV-2 specific primer pair has a length of about 390 bp, the amplicon generated by the DENV-3 specific primer pair has a length of about 731 bp and the amplicon generated by the DENV-4 specific primer has a length of about 226 bp.

7. The method of any one of claims 3 to 6, wherein prior to step a) the live, attenuated dengue virus is grown in Vero cells.

8. The method of any one of claims 1 to 7, wherein the virus nucleic acids are isolated virus RNA and step b) comprises a step of reverse transcription of the isolated virus RNA in the presence of a reverse transcriptase using a reverse primer.

9. The method of any one of claims 1 to 8, wherein steps b) and c) are carried out in the form of a one-step RT-PCR reaction.

10. The method of any one of claims 1 to 9, wherein the final concentration in the reaction mix in step c) of the forward primer and the reverse primer for each of the primer pairs (i) to (iv) is about 0.3 to 0.5 µM, more preferably 0.4 µM.

11. The method of claim 9 or 10, wherein the amplifying step c) is carried out in a thermocycling process at +50°C for 20 min; +95°C for 2 min; and multiple cycles of +95°C for 30 s; +66°C for 30s and +72°C for 1 min.

12. The method according to any one of claims 1 to 11, wherein step d) includes a gel electrophoresis in an agarose gel or a capillary electrophoresis.

13. The method according to claim 12, insofar the separating step d) includes an agarose gel and the run conditions for the agarose gel include the use of a 1x TAE buffer and a voltage of about 90 to 110 V.

14. The method according to any one of claims 1 to 13, wherein step e) comprises visualization of the amplicons by staining, preferably ethidium bromide staining is used.

15. The method according to any one of claims 1, 2, and 5 to 14, wherein said sample is from a dengue disease patient.

16. The method of any one of claims 1 to 14, wherein the method does not comprisesequencing of the generated amplicon(s).

17. Use of the method according to any one of claims 1 to 14 and 16 in the quality control of vaccines containing live, attenuated dengue virus.

18. Use of the method according to any one of claims 1, 2, 5, 6, and 8 to 16 in the diagnosis of a dengue virus infection in a patient sample.

19. A kit for detecting the presence of dengue virus serotype in a sample containing at least one dengue virus comprising
(i) a dengue serotype 1 (DENV-1) specific primer pair, wherein the forward primer comprises the nucleotide sequence set forth in SEQ ID NO: 1 or a variant thereof having at least 85% sequence identity thereof and the reverse primer comprises the nucleotide sequence set forth in SEQ ID NO: 2 or a variant thereof having at least 85% sequence identity thereof;
(ii) a dengue serotype 2 (DENV-2) specific pair, wherein the forward primer comprises the nucleotide sequence set forth in SEQ ID NO: 3 or a variant thereof having at least 85% sequence identity thereof and the reverse primer comprises the nucleotide sequence set forth in SEQ ID NO: 4 or a variant thereof having at least 85% sequence identity thereof;
(iii) a dengue serotype 3 (DENV-3) specific primer pair, wherein the forward primer comprises the nucleotide sequence set forth in SEQ ID NO: 5 or a variant thereof having at least 85% sequence identity thereof and the reverse primer comprises the nucleotide sequence set forth in SEQ ID NO: 6 or a variant thereof having at least 85% sequence identity thereof; and/or
(iv) a dengue serotype 4 (DENV-4) specific primer pair, wherein the forward primer comprises the nucleotide sequence set forth in SEQ ID NO: 7 or a variant thereof having at least 85% sequence identity thereof and the reverse primer comprises the nucleotide sequence set forth in SEQ ID NO: 8 or a variant thereof having at least 85% sequence identity thereof.

20. Use of the kit of claim 19 in the quality control of one or more vaccines containing live, attenuated dengue virus.

21. Use of the kit of claim 19 in the diagnosis of a dengue virus infection in a patient sample.

22. A quality control method for vaccines containing live, attenuated dengue virus comprising performing the method according to any one of claims 1 to 14 and 16 and at least one further method selected from the group consisting of immunofocus assay, attenuation check, quantitative determination of human serum albumin (HSA) monomer, visual inspection, determination of reconstitution time or resuspendability of lyophilisates, sterility test, test for bacterial endotoxins, determination of host cell DNA, determination of pH, colorimetric determination of water, determination of osmolality, determination of the content of one or more excipient(s).

23. The quality control method of claim 22, comprising performing the method according to any one of claims 1 to 14 and 16 and an attenuation check method comprising the steps of:
d) purifying flavivirus nucleic acids from said formulation;
e) preparing double-stranded DNA templates from said flavivirus nucleic acids;
f) amplifying said viral nucleic acids of a) or said double-stranded DNA templates of b) by polymerase chain reaction (PCR) by using a primer pair comprising sequences specific for a flavivirus sequence comprising the at least one attenuation locus such that PCR products comprising the at least one attenuation locus are generated;
d) sequencing said PCR products by next generation sequencing by synthesis selected from HiSeq sequencing, MiSeq sequencing and pyrosequencing; and
e) determining the nucleotide sequence at the at least one attenuation locus.

24. The quality control method of claim 23, wherein the live, attenuated flavivirus is a dengue virus comprising three attenuation loci at positions 57, 2579 and 5270 of the nucleotide sequence of dengue virus type 2 according to SEQ ID NO: 10 and the primer pair of step c) is selected from the group consisting of:
i) a forward primer comprising SEQ ID NO: 17, or a variant thereof having at least 85% sequence identity thereof and a reverse primer comprising SEQ ID NO: 18, or a variant thereof having at least 85% sequence identity thereof such that a first PCR product having a length of about 310 bp to about 330 bp and comprising the first attenuation locus is generated;
ii) a forward primer comprising SEQ ID NO: 19, or a variant thereof having at least 85% sequence identity thereof and a reverse primer pair comprising SEQ ID NO: 20, or a variant thereof having at least 85% sequence identity thereof such that a second PCR product having a length of about 320 bp to about 350 bp and comprising the second attenuation locus is generated; and
iii) a forward primer comprising SEQ ID NO: 21, or a variant thereof having at least 85% sequence identity thereof and a reverse primer comprising SEQ ID NO: 22, or a variant thereof having at least 85% sequence identity thereof such that a third PCR product having a length of about 400 bp to about 430 bp and comprising the third attenuation locus is generated.

25. The quality control method of claim 22, comprising performing the method according to any one of claims 1 to 14 and 16 and quantitative determination of human serum albumin (HSA) monomer.

26. The quality control method of claim 25, wherein the method for quantitative determination of human serum albumin (HSA) monomer comprises the steps of:
(a) applying about 5 µL to about 15 µL of the liquid composition on a size-exclusion chromatography column, wherein the column material comprises diol residues coupled to a silica gel;
(b) performing size-exclusion chromatography at a flow rate of about 0.7 to about 0.9 mL/min with a mobile phase comprising about 0.08 to about 0.12 M phosphate buffer with about 0.08 M to about 0.12 M sodium sulfate at a pH value from about 6.8 to about 7.0;
(c) detecting the UV absorption of the HSA monomer at the outlet of the column; and
(d) calculating the amount of HSA monomer from the area under the curve (AUC) of the detected signal.
